# EUROPEAN PATENT SPECIFICATION

(11) **EP 2 177 909 B1**
(45) Date of publication and mention of the grant of the patent: **24.04.2013**
(21) Application number: 09172180.3
(22) Date of filing: 05.10.2009
(51) Int. Cl.: B82Y 15/00, G01N 33/543, G01N 33/538

(54) **Solid support with enhanced density of signal material and method of detecting target material using the same**
Fester Träger mit verbesserter Signalmaterialdichte und Verfahren zur Erkennung von Zielmaterial damit
Support solide avec densité améliorée de matériau de signal et procédé de détection de matériau cible l'utilisant

(30) Priority: 15.10.2008 KR 20080101210
(43) Date of publication of application: 21.04.2010
(73) Proprietor: Samsung Electronics Co., Ltd., Suwon-si, Gyeonggi-do, 443-742 (KR)
(72) Inventor: Lee, Beom Seok, Hwaseong-si Gyeonggi-do (KR); Lee, Jeong Gun, Seoul (KR); Oh, Byung Keun, Seoul (KR); Choi, Jeong Woo, Seoul (KR)
(74) Representative: Grünecker, Kinkeldey, Stockmair & Schwanhäusser

(56) References cited:
- WO-A1-01/44812
- WO-A2-2006/125050
- MCCREERY T: "Digoxigenin labeling." MOLECULAR BIOTECHNOLOGY APR 1997, vol. 7, no. 2, April 1997 (1997-04), pages 121-124, XP002564660 ISSN: 1073-6085

## Description

### BACKGROUND

### 1. Field

One or more embodiments relate to a solid support comprising a plurality of labeled nucleic acid molecules, and a method of detecting a target material using the solid support.

### 2. Description of the Related Art

Various methods of detecting a target material are known. A chemiluminescent detection method of detecting a target material such as DNA, RNA, or a protein by employing a chemiluminescent compound as a signal generating compound or "label" is an example of these methods. The chemiluminescent detection method can be performed by employing a chemiluminescent compound to label a target material or a compound that is specifically linked to the target material. Also, the chemiluminescent detection method can be performed by employing an enzyme that catalyzes a chemiluminescent reaction of a chemiluminescent compound that is coupled to the target material or with a catalyst that functions similar to the enzyme. The chemiluminescent detection method is safe compared to radioimmunoassay which employs radioactive substances, and can be used in various ways. A probe which is suitable for use in an assay to detect a target material is labeled with haptens such as biotin, fluorescein, or digoxygenine. These haptens are linked to a ligand or antibody which is conjugated to an enzyme such as horseradish peroxidase (HRP) and alkaline phosphatase (AP). The labeled enzyme is detected using a substrate, which generates detectable signals such as optically detectable signals by the action of the enzyme on the substrate.

A target material may be detected using a signal material that is linked to a probe and generates a detectable signal. One or more examples of the disclosure are to enhance sensitivity of the assay.

WO 01/44812 A1 relates to a process for preparing a micro-particle reagent assemblage capable of detecting a plurality of analytes in a sample as well as a method for the simultaneous determination of multiple distinct analytes using said micro-particle reagent assemblage. The process comprises immobilizing a first specific micro-identification tag and a first affinity ligand, specific for a given first analyte in the sample onto the surface of microparticles, to form a micro-particle subset. A plurality of micro-particle subsets are formed, which plurality of micro-particle subsets are then mixed to produce the micro-particle reagent assemblage, wherein each affinity ligand is identifiable by the immobilized micro-identification tag. The detection method comprises admixing a sample with said micro-particle reagent assemblage; incubating the sample with the reagent assemblage under conditions conducive to complex formation between an analyte comprised in the sample and a corresponding affinity ligand; and determining the present of distinct analytes by correlating the formation of an analyte/affinity ligand complex with the micro-identification tag of the corresponding micro-particle subset.

WO 2006/125050 A2 relates to a detection method of chemical detection through signal amplification from a reporter reagent capable of selectively binding to the target chemical of interest. Said method utilizes a detection reagent containing a streptavidin-biotin complex or a particle that has been derivatized to include on its surface selective binding compounds that specifically bind to the target chemical species and a large number of copies of a pre-selected reporter moiety. According to the detection method of WO 2006/125050 A2, a capture probe comprising at least one binding compound that specifically binds to the target analyte, as well as the above described detection reagent are incubated under conditions effective to allow a complex formation between the capture probe, the target analyte and the detection reagent; separating the complex from unbound detection reagent; selectively releasing at least a portion of the reporter moieties from the complex; and determining the presence or absence of the target analyte by analyzing the presence or absence of reporter moieties.

McCreery, Tom: "Digoxigenin labeling", Molecular Biotechnology, vol. 7, no. 2, April 19, 1997, pages 121-124 is a review regarding digoxigenin labeling for nonradioactive detection of nucleic acids and proteins, said review disclosing a variety of methods for labeling DNA with digoxigenin, such as end-labeling, Nick translation PCR using digoxigenin-11-dUTP, 3'-end-labeling using terminal transferase or 3'-tailing using a terminal transferase, as well as applications for digoxigenin-labeled probes and detection methods thereof.

### SUMMARY

One or more embodiments include a solid support as claimed.

One or more embodiments include a method of detecting a target material using a solid support as claimed.

Additional aspects will be set forth in part in the description which follows and, in part, will be apparent from the description, or may be learned by practice of the embodiments.

### BRIEF DESCRIPTION OF THE DRAWINGS

These and/or other aspects will become apparent and more readily appreciated from the following description of the embodiments, taken in conjunction with the accompanying drawings of which:
FIG. 1 is a diagram illustrating a labeled nucleic acid molecule which may be bound to a solid support according to an exemplary embodiment, wherein the labeled nucleic acid molecule includes a nucleotide residue which is bound to a first linking moiety, and a method of manufacturing the labeled nucleic acid molecule according to an exemplary embodiment;
FIG. 2 is a diagram illustrating an example of a solid support which contains a target capturing molecule and a labeled nucleic acid molecule, in which the labled nucleic acid molecule includes a nucleotide residue which is coupled to a first linking moiety, and a method of manufacturing the solid support according to an exemplary embodiment;
FIG. 3 is a diagram illustrating a method of detecting a target material using a solid support according to an exemplary embodiment;
FIG. 4 is a graph of HRP activity measured from a DIG-labeled nucleic acid with respect to the number of gold particles;
FIG. 5 is a graph of HRP activity of gold particles on which an HRP labeled anti-DIG antibody is directly immobilized and gold particles on which a DIG-labeled nucleic acid is immobilized and which are linked with an HRP labeled anti-DIG antibody; and
FIG. 6 is a diagram illustrating PSA assay results using a first solid support, with respect to the concentration of PSA in a sample.

In Figures, the symbols "DIG," "ss-DNA," "HRP," and "PSA," each stand for "digoxygenin," "single stranded DNA," "horseradish peroxidase," and "prostate specific antigen," respectively

### DETAILED DESCRIPTION

An exemplary embodiment provides a solid support according to claim 1, comprising a plurality of labeled nucleic acid molecule.

The first linking moiety may be a signal material that emits a detectable signal. For example, the first linking moiety may be selected from fluorescent materials and radioactive materials, but is not limited thereto. In addition, the first linking moiety may be a material that generates an electric signal, a fluorescent signal, a radioactive signal, or a surface acoustic wave.

The first linking moiety may be a hapten molecule, a ligand that is specifically linked or a molecule having an anti-ligand relationship. For example, the first linking moiety may be digoxygenin (DIG), dinitrophenol (DNP) or biotin; or anti-DIG antibody, anti-DNP antibody, avidin or streptavidin.

The first linking moiety may be linked to a first linking partner molecule. The first linking partner may be a material that may specifically bind to the first linking moiety. For example, the first linking partner may be an antibody or its fragments such as Fab, a nucleic acid or oligonucleotide, a ligand or an anti-ligand.

For example, when a first linking moiety is one of digoxygenin (DIG), dinitrophenol (DNP), or biotin, the first linking partner may be anti-DIG antibody, anti-DNP antibody, or avidin or streptavidin, respectively.

The first linking partner may be labeled with an enzyme. The enzyme may catalyze a reaction for converting a substrate into a material capable of generating a detectable signal. For example, the enzyme may be horseradish peroxydase (HRP), beta-galactosidase, glucuronidase, or alkaline phosphatase (AP), but is not limited thereto.

The length of the labeled nucleic acid molecule is in a range of about 50 bp to about 300 bp. The labeled nucleic acid molecule has a region (sometimes referred to as "spacer region" for convenience) which is free of the first linking moiety. The spacer region covers from the 5' end or 3' end to the about 30^{th} nucleotide residue. The labeled nucleic acid molecule has a first linking moiety which binds to a nucleotide residue between position 30 (from the 5' end or 3' end of the labeled nucleic acid molecule) to the other end of the molecule. The region is sometimes also referred to as a "tail region," for convenience. In the tail region, a plurality of nucleotide residues bind to a first linking moiety.

A nucleic acid molecule may be selected from the group consisting of DNA, RNA, and a chimera molecule of DNA and RNA. Also, the nucleic acid molecule may be a double-stranded or single-stranded nucleic acid molecule.

The number of nucleotide residues having the first linking moiety is 2 or more, 5 or more, 10 or more, or 20 or more per one labeled nucleic acid molecule. The nucleic acid molecule having nucleotide residues binding to the first linking moiety may be prepared by employing nucleotides bound to the first linking moiety in a nucleic acid polymerization or extension reaction. Alternatively, a non-reformed nucleotide is added during a nucleic acid polymerization or extension reaction and then reformed.

For example, a nucleic acid polymerization may be performed employing a double-stranded DNA having a 5' overhang in the presence of the nucleotide having the first linking moiety (for example, DIG-11-dUTP) and DNA polymerase (for example, Klenow). Alternatively, a labeled RNA may be obtained by polymerizing in the presence of a template RNA, a nucleotide having a first linking moiety (For example, DIG-11-dUTP), and an RNA polymerase (for example, T7/SP6 RNA polymerase). Alternatively, a nucleic acid molecule labeled with a nucleotide having a first linking moiety may be obtained by performing PCR in the presence of a nucleotide having a first linking moiety (for example, DIG-11-dUTP). Alternatively, the labeling may be performed by causing a nick translation in the presence of a nucleotide having a first linking moiety (for example, DIG-11-dUTP), a DNA polymerase and DNase. However, the method is not limited to these examples and may be any known addition method.

The labeled nucleic acid molecule is linked to the solid support through a 3' end or 5' end of the labeled nucleic acid. The labeled nucleic acid molecule has a spacer region of 30 bp, at its 3' or 5' end, where no first linking moiety is added, and the labeled nucleic acid is linked to the solid support through the free end of the spacer region.

A method for coupling or immobilizing a nucleic acid molecule to a solid support is known. For example, an end of a nucleic acid molecule is modified using a functional group such as -SH or an amino group; or a material including such functional group, and then, the nucleic acid molecule is allowed to react with a surface of a solid support that is reactive with the functional groups (for example, gold particle surface, or aldehyde or activated ester group) or a surface of a solid support to which a reactive group is introduced, thereby linking the nucleic acid to the solid support. For example, a nucleic acid molecule that has a 3' or 5' end reformed as a material having -SH is reacted with gold particles, so that the nucleic acid molecule is immobilized on a surface of gold particles.

The solid support may be nanoparticles or microparticles. For example, the solid support may have a diameter or a length of one side surface of a cross-section thereof in a range of about 1 nm to about 1000 µm. The solid support may have various shapes. For example, the solid support may be spherical, flat, or cylindrical, or have a bead-like shape. The solid support may be made of a various material. For example, the solid support may be formed of a material selected from gold, polystyrene, polymethylmethacrylate (PMMA), or magnetic particles.

According to an exemplary embodiment, the solid support may be gold nanoparticles, the nucleotide having the first linking moiety of the labeled nucleic acid may be digoxygenin (DIG)-11-dUTP, and the first linking partner may be an anti-DIG antibody labeled with horseradish peroxydase (HRP). For DIG-11-dUTP, DIG may be directly connected to a fifth carbon site of uridine of dUTP through 11 position of DIG, or indirectly connected to a fifth carbon site of uridine of dUTP through 11 position of DIG through a spacer. For example, DIG-11-dUTP may have a structure shown below:

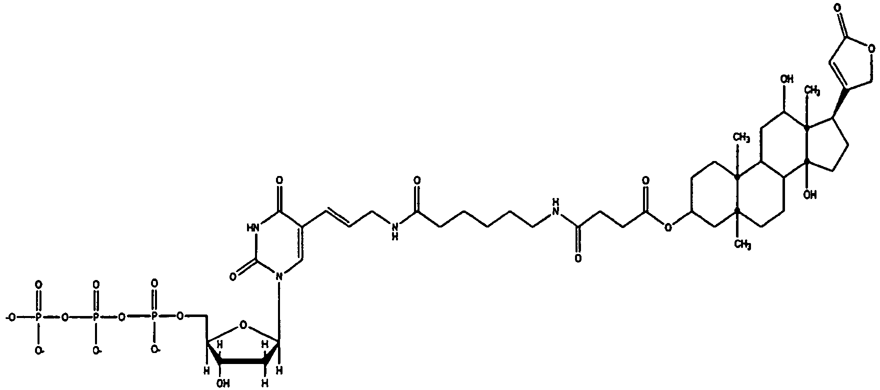

The solid support is further linked to a first target capture molecule that can bind to a target material.

The first target capture molecule may be a molecule that binds specifically or non-specifically a target material. For example, the first target capture molecule may be an antibody that binds to a target material, a nucleic acid that binds to a target material, a ligand that binds to a target material, or an anti-ligand that binds to a target material. The target material may be a material to be assayed. For example, the target material may be a macromolecule such as a polypeptide, a nucleic acid, a carbohydrate, or a small molecule.

The solid support immobilized with the labeled nucleic acid may be used as a probe for detecting a target molecule.

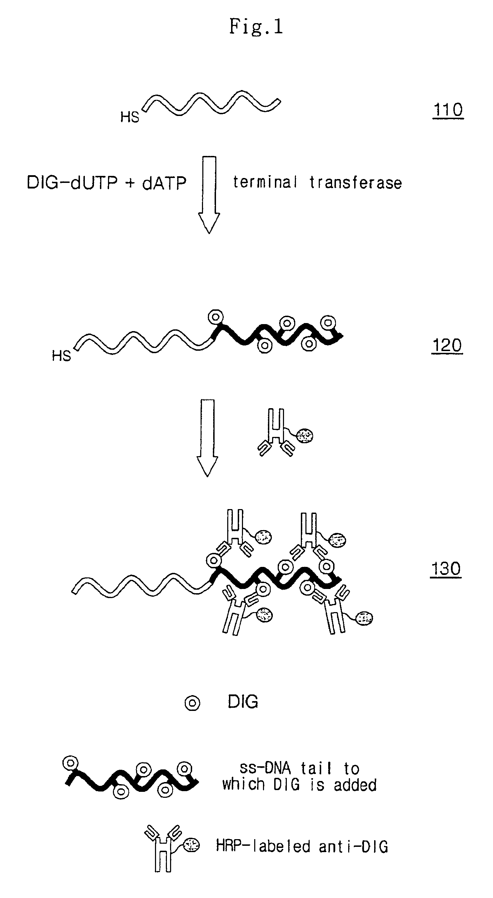

FIG. 1 is a diagram illustrating a labeled nucleic acid molecule which can be immobilized to a solid support according to an exemplary embodiment, wherein the labeled nucleic acid molecule includes a nucleotide residue to which a first linking moiety binds. It also depicts a diagram showing a method of manufacturing the labeled nucleic acid molecule according to an exemplary embodiment. Referring to FIG. 1, a reference numeral 110 designates a single-stranded nucleic acid (for example, DNA; "ssDNA") in which 5'-OH of ribose of the 5' end is substituted with - SH. The -SH may have a spacer group (for example, an alkyl group including a C1-C10 alkyl group). In this case, 5'-OH may be substituted with -SH through the spacer group. A reference numeral 120 designates an ssDNA to which DIG is added, and a reference numeral 130 designates a nucleic acid labeled with HRP through DIG. A method of manufacturing the labeled nucleic acid molecule according to an exemplary embodiment will now be described briefly. First, the single-stranded DNA 110 in which the 5'-OH of ribose of a 5' end is substituted with -SH is reacted in the presence of DIG-11-dUTP, dATP and terminal transferase, thereby producing the ssDNA 120 having the 3' end region that has a tail to which DIG is added. Then, the ssDNA 120 having the 3' end region that has a tail to which DIG is added is allowed to bring in contact with a HRP-labeled anti-DIG antibody, thereby producing a ssDNA 130 that is labeled with HRP through a DIG-anti-DIG antibody complex. The HRP-labeled anti-DIG antibody corresponds to the first linking partner.

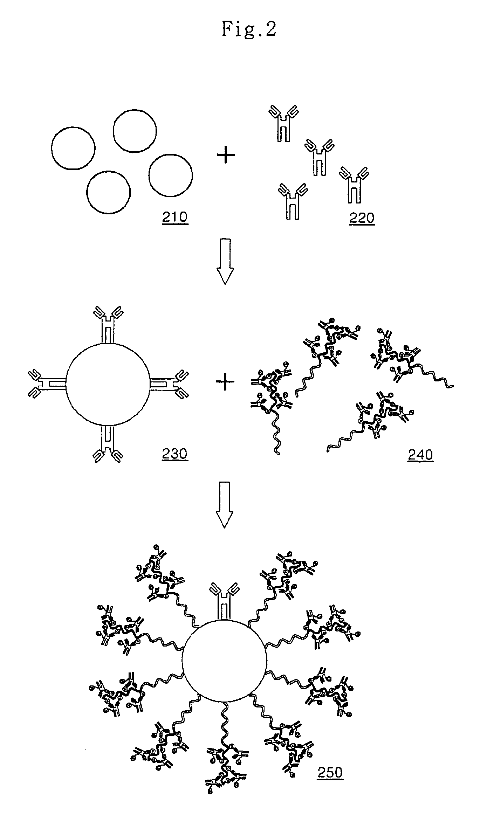

FIG. 2 is a diagram illustrating an example of a solid support linked with a first target linking molecule and a labeled nucleic acid molecule. FIG. 2 also shows a method of manufacturing the solid support according to an exemplary embodiment. Referring to FIG. 2, a reference numeral 210 designates a solid support, a reference numeral 220 designates a first target capture molecule (for example, an antibody that specifically binds to a target molecule), a reference numeral 230 designates a solid support that is linked with the first target capture molecule 220, a reference numeral 240 designates a labeled nucleic acid molecule which has a first linking moiety (for example, ssDNA labeled with HRP through a DIG-anti-DIG antibody complex illustrated in FIG. 1), and a reference numeral 250 designates a solid support on which the first target capture molecule 220 and the labeled nucleic acid moleculre 240 are immobilized. A method of manufacturing the solid support 250 according to an exemplary embodiment will now be described briefly. First, the first target capture molecule 220 that specifically binds to a target molecule is immobilized on the solid support 210 (for example, gold nanoparticles), thereby obtaining the solid support 230 on which the first target capture molecule 220 is immobilized. Then, the labeled nucleic acid molecule 240 (for example, ssDNA labeled with HRP through a DIG-anti-DIG antibody complex illustrated in FIG. 1) is immobilized on the surface of the solid support 230 that has the first target capture molecule 220, to give the solid support 250 that has the first target capture molecule 220 and the labeled nucleic acid molecule 240.

Another example of the present application relates to a kit for detecting a target material, wherein the kit includes a first solid support as described above. The first solid support and the target material have been described above.

The kit may further include a second solid support linked with a second target capture molecule that is linked to the target material.

The first target capture molecule and the second target capture molecule may have different binding specificities. For example, the first target capture molecule and the second target capture molecule may binds to different sites (e.g., different epitomes of an antigen) of the same target molecule.

The second solid support may include nanoparticles or microparticles. For example, the second solid support may have a diameter or a length of one side surface of a cross-section thereof in a range of about 1 nm to about 1000 µm. The second solid support may have various shapes. For example, the second solid support may be spherical, flat, or cylindrical, or have a bead-like shape. The second solid support may be formed of various materials. For example, the second solid support may be formed of a material selected from gold, polystyrene, polymethylmethacrylate (PMMA), magnetic particles.

The second target capture molecule may be linked to the second solid support using a known method which may vary according to the second target capture molecule selected. For example, if the second target capture molecule is a nucleic acid, an end of the nucleic acid is modified with a functional group such as -SH or amino group, or a material including these functional group, and then, the nucleic acid is allowed to be in contact with a surface of a solid support having a reactive group (for example, gold particle surface or aldehyde or activated ester group) or a surface of a solid support to which a reactive group is introduced, thereby coupling the second target capture molecule to the second solid support. For example, a nucleic acid molecule that has a 3' or 5' end modified to have -SH may be coupled to gold particles, so that the nucleic acid molecule is immobilized on a surface of the gold particles.

The second solid support may or may not be linked to the labeled nucleic acid.

The kit may include a microfluidic apparatus including a chamber including the first solid support.

The kit may include a specification (an insert) describing a protocol of detecting a target material including linking the target material to the first target linking molecule linked to the first solid support and measuring a signal generated by the labeled nucleic acid, or a method of detecting a target material in a sample according to another exemplary embodiment.

Another exemplary embodiment provides a method of detecting a target material in a sample, wherein the method includes: bringing the sample into contact with a first solid support as described above and a second solid support linked with a second target capture molecule as described above; isolating a composite of the target material and the first and second solid supports by centrifuging or using a magnet; and detecting a signal generated by the isolated composite.

The step of bringing the sample into contact with a first solid support as described above and a second solid support linked with a second target capture molecule as described above may be conducted under conditions which may vary according to the first and second solid supports selected, a target material to be detected, and a target capture molecule selected. For example, this contact process may be performed in a buffer having an appropriate pH and salt concentration, for example in a PBS or Tris buffer. The contact conditions would be obvious to one having ordinary skill in the art. The first and second solid supports have been described above.

As a result of the contact process, the target material in the sample binds to the first target capture molecule of the first solid support and the second target capture molecule of the second solid support, thereby forming a composite of the target material, and the first and second solid supports. A signal generated by the composite may be measured in a state in which the composite is isolated or is not isolated. For example, the signal may be measured when the composite is isolated.

The method of detecting a target material in a sample may include the step of isolation of a composite of the target material and the first and second solid supports. The isolation may be performed using a known method. For example, the isolation may be performed by centrifuging or using magnetism, if at least one of the first and the second sold support is made of a magnetic material.

The method of detecting a target material in a sample may include the step of detection of a signal generated by the resulting composite. The first linking moiety of the first solid support contained in the composite may be a signal material that emits a detectable signal, and the detection process may be performed by measuring the detectable signal generated by the signal material. The signal material may be a optical (e.g., fluorescent) material or a radioactive material.

Also, the first linking moiety of the first solid support contained in the composite may be linked to a first linking partner and the detection process may be performed by measuring a signal generated by the first linking partner. For example, the detection process may be performed by directly measuring a signal generated by the first linking partner. Alternatively, an enzyme which is conjugated to the first linking partner as a label is reacted with a substrate of the enzyme, which generates a detectable signal, and then the signal is measured.

The enzyme may be, for example, an enzyme that is used in a chemiluminescent method. For example, the enzyme may be selected from horseradish peroxidase (HRP), beta-galactosidase, glucuronidase and alkaline phosphatase (AP), but is not limited thereto.

In the method of detecting a target material in a sample, the first solid support may be linked with the first target capture molecule, and the first target capture molecule may have different binding specificities from the second target capture molecule.

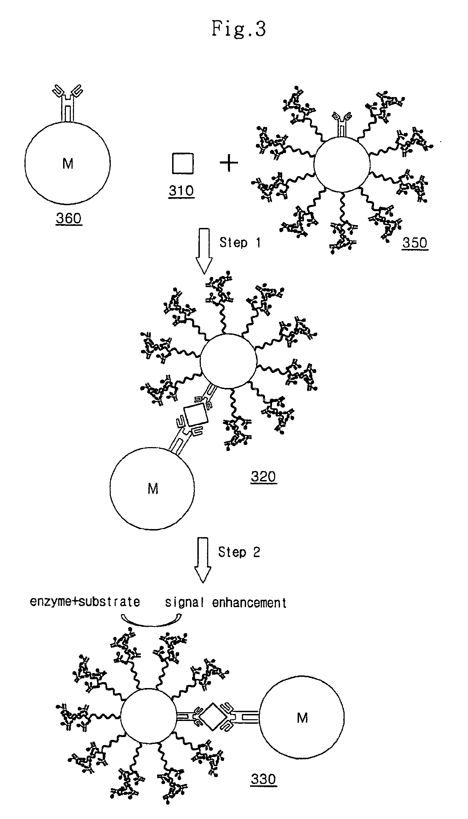

FIG. 3 is a diagram illustrating a method of detecting a target material using a solid support according to an exemplary embodiment. First, a sample which is to be assayed for a target molecule 310 (e.g., prostate specific antigen (PSA)), a second solid support (for example, a magnetic particle) 360 on which a second target capture molecule (for example, an anti-PSA antibody) is immobilized, and a first solid support 350 that is linked with a first target capture molecule and a labeled nucleic acid molecule are brought into contact to obtain a composite 320 of first solid support-target molecule-second solid support. In this embodiment, the first solid support 350 is a gold particle on which ssDNA labeled with HRP through a DIG-anti-DIG antibody complex (FIG. 1) and an anti-PSA antibody that recognizes a different epitope of PSA from the second target capture molecule are immobilized.

Then, the composite 320 may be separated from a reaction mixture using a magnetic force, and then, for example, a washing process is performed, and a signal generated from the first solid support is detected. The signal detection may be performed by detecting a signal generated by, for example, the first linking moiety immobilized on the first solid support or a first linking partner linked to the first linking moiety. For example, if the first linking partner is HRP, the first linking partner is reacted with 3',5,5'-tetramethylbenzidine (TMB, (a substrate of HRP) to produce a chromogenic substrate and an optic signal generated by the chromogenic substrate is measured. The reaction conditions for HRP and TMB are known and can be determined by one skilled in the art.

Another exemplary embodiment provides a labeled nucleic acid molecule having nucleotide residues bound to a first linking moiety, wherein the first linking moiety may be linked to a first linking partner.

The first linking moiety may be selected from digoxygenin (DIG), dinitrophenol (DNP), biotin, or fluorescein.

The first linking partner may be specifically or non-specifically linked to the first linking moiety. For example, the first linking partner may be an antibody or nucleic acid that is specifically linked to the first linking moiety. The first linking partner may be selected from anti-DIG, anti-DNP, avidin or streptavidin.

The first linking partner may be labeled with an enzyme. The enzyme may be selected from horseradish peroxidase (HRP), beta-galactosidase, glucuronidase or alkaline phosphatase (AP), but is not limited thereto.

The length of the labeled nucleic acid is not limited. For example, the length of the labeled nucleic acid molecule may be in a range of about 50 bp to about 300 bp. The labeled nucleic acid may have a spacer region which is free of first linking moiety within a predetermined length of sequence from a 5' end or 3' end of the labeled nucleic acid. For the labeled nucleic acid molecule, the nucleotide residues ranging from the 5' or 3' end of the molecule to 30^{th} position may not coupled to a first linking moeity. For example, nucleotide residues bound to the first linking moiety may be present between about 30^{th} position to the other end of the molecule. This region of the labeled nucleic acid molecule, where a nucleotide residue is bound to the first linking moiety is sometimes also referred to as a "tail region" for convenience.

A nucleic acid may be selected from the group consisting of DNA, RNA, and a chimera molecule of DNA and RNA. Also, the nucleic acid may be a double-stranded or single-stranded nucleic acid.

The number of nucleotides having the first linking moiety, in the labeled nucleic acid may be 2 or more, 5 or more, 10 or more, or 20 or more per labeled nucleic acid molecule.

The present invention will be described in further detail with reference to the following examples.

### Example 1: Synthesis of DIG-labeled nucleic acid and gold particle on which the labeled nucleic acid is immobilized

In the present example, a single-stranded DNA was reacted with DIG-11-dUTP in the presence of a terminal deoxynucleotidyl transferase, thereby producing a single-stranded DNA having a 3' end region to which at least one DIG was added. Herein, the single-stranded DNA having a 3' end region to which at least one DIG was added constitutes a labeled DNA. The labeled DNA was immobilized on gold particles, thereby producing gold particles on which the labeled DNA was immobilized.

First, a single-stranded DNA having a sequence set forth in SEQ ID NO.1 in which -SH is linked to a hydroxyl group of a 5' end (5'-SH-TTGGGTAACGCCAGGGTTTTCCCA GTC-3', Genotech) was produced. Then, the single-stranded DNA was reacted with DIG-11-dUTP and dATP in the presence of a terminal deoxynucleotidyl transferase (TdT) at a temperature of 37°C for 15 minutes, thereby synthesizing a nucleic acid having a 3' end region that is labeled with DIG. TdT is an enzyme that catalyzes a reaction for adding a nucleotide to a 3' end of a DNA molecule. The reaction was performed using a commercially available DIG Oligonucleotide Tailing Kit (Roche Co.) according to a specification of the DIG Oligonucleotide Tailing Kit. In the reaction, concentrations of the single-stranded DNA, DIG-11-dUTP, and dATP were about 100 pM, about 1 mM, and about 10 mM, respectively. The concentration of TdT used was 400U. As a result, about 50 bp of DNA sequence was added to the 3' end, and the number of DIG-11-dUTP added was assumed to be about 5, according to the protocol provided by the manufacturer.

Then, the labeling of the synthesized nucleic acid molecule was confirmed using an anti-DIG antibody labeled with HRP. First, 100 µM of the synthesized nucleic acid was reacted with 1.1x10¹⁰ of gold particles (BBinternational Co., Gold Colloid: 80 nm) having an average diameter of about 80 nm, thereby immobilizing the synthesized nucleic acid on gold particles through-SH of the 5' end.

The gold particles on which the synthesized nucleic acid was immobilized were reacted with an anti-DIG antibody labeled with HRP (Roche Co.), thereby forming a composite of DIG immobilized on gold particles and the anti-DIG antibody. Then, the composite was isolated using a centrifuge at a temperature of 4°C and a revolution speed of about 13000 rpm, and washed at least 4 times to prevent a reaction caused by HRP at supernatant. 3,3',5,5'-tetramethylbenzidine (TMB) that constitutes a substrate was added to the isolated composite and reacted for 10 minutes. Then, a trace amount of 2M H₂SO₄ was added to the reaction solution in order to stop the reaction, and absorbance of the reaction solution was measured at a wavelength of 450 nm to measure a level of HRP activity. Meanwhile, gold particles on which DNA having a sequence set forth in SEQ ID NO:1 to which DIG was not added were used as a control group.

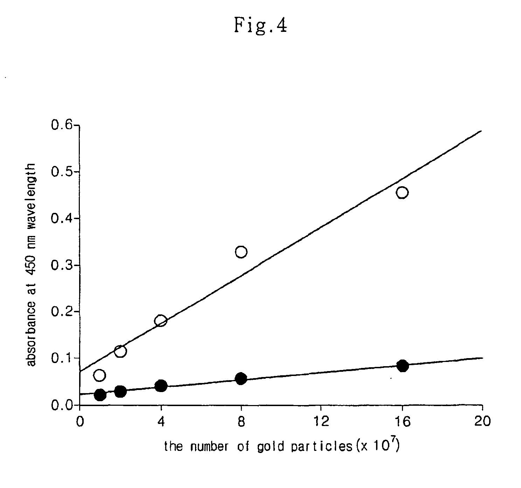

As a result, for an experimental group (empty circles of FIG. 4), as the number of gold particles increased, HRP activity increased. Accordingly, it can be seen that the 3' end region of the nucleic acid was labeled with DIG For a control group (solid circles of FIG. 4), as the number of gold particles increased, HRP activity was not significantly changed.

FIG. 4 is a graph of HRP activity measured from the DIG-labeled nucleic acid with respect to the number of gold particles.

According to Example 1, a nucleic acid is labeled with at least one DIG so that the density of DIG per nucleic acid molecule can be increased. Also, at least one labeled nucleic acid is immobilized on gold particles so that the density of DIG per gold particle can be further increased. Accordingly, for an assay using a HRP labeled anti-DIG, use of DIG-labeled nucleic acid and particles on which the DIG-labeled nucleic acid is immobilized leads to an increase in assay sensitivity.

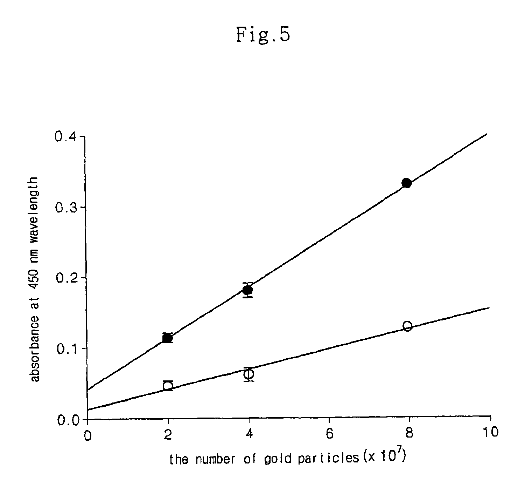

FIG. 5 is a graph of HRP activity of gold particles on which an HRP labeled anti-DIG antibody was directly immobilized and gold particles on which a DIG-labeled nucleic acid was immobilized and which are linked with the HRP labeled anti-DIG antibody.

Referring to FIG. 5, the gold particles on which the HRP labeled anti-DIG antibody was directly immobilized (control group) were obtained by reacting gold particles with an HRP labeled anti-DIG antibody (Roche Co.) at room temperature for 30 minutes so that the labeled anti-DIG antibody was physically linked to the gold particles. The gold particles on which the DIG-labeled nucleic acid was immobilized and which were linked with the HRP labeled anti-DIG antibody (experimental group) was produced as described above. TMB that constitutes a substrate was added to the control group and the experimental group and reacted for 10 minutes. 2M H₂SO₄ was added to the reaction solution in order to stop the reaction and absorbance of the reaction solution was measured at a wavelength of 450 nm to measure the level of HRP activity.

As illustrated in FIG. 5, the experimental group (solid circles) showed higher enzyme activity and larger increase in enzyme activity with respect to the number of gold particles of the experimental group (solid circles) than the control group (empty circles). Accordingly, it can be seen that HRP enzyme activity, that is, a detection signal can be amplified by immobilizing the labeled nucleic acid on the solid support.

### Example 2: Synthesis of gold particles on which DIG-labeled nucleic acid and first target linking molecule are immobilized

In the present example, 25 ng of rat anti-PSA antibody was reacted with 1x10⁹ of gold particles in order to produce gold particles on which the rat anti-PSA antibody was immobilized. In this case, the concentration of the immobilized rat anti-PSA antibody was determined such that the rat anti-PSA antibody was immobilized on half the entire surface of the gold particles. This is to guarantee a space for the DIG-labeled nucleic acid on the surface of the gold particles.

Such a concentration was determined using the following method. First, gold particles were reacted with various concentrations of the rat anti-PSA antibody in order to produce gold particles on which the rat anti-PSA antibody was immobilized, and then an agglomeration test was performed on the obtained respective particles using NaCl. According to an agglomeration test, as the amount of an antibody used in a reaction is smaller, more agglomeration occurs, and as the amount of an antibody is so high that the surfaces of gold particles are completely surrounded, agglomeration of gold particles due to salts does not occur. In this case, there is no space for DNA to react. Accordingly, in this experiment, a concentration of an antibody corresponding to a case in which about half of gold particles agglomerate was used.

Then, the gold particles on which the rat anti-PSA antibody was immobilized were reacted with the DIG- labeled nucleic acid produced according to Example 1 at room temperature for 15 minutes, thereby immobilizing the DIG-labeled nucleic acid on other portion of each gold particle, on which the rat anti-PSA antibody was immobilized, through -SH of a 5' end. The gold particles on which the DIG-labeled nucleic acid was immobilized were reacted with an HRP-labeled anti-DIG antibody (Roche Co.), thereby producing a composite of DIG immobilized on gold particles and the HRP-labeled anti-DIG antibody. Then, the composite was isolated using a centrifuge at a temperature of 4°C at a revolution rate of about 13000 rpm and washed at least 4 times to prevent a reaction caused by HRP at supernatant. TMB that constitutes a substrate was added to the isolated composite and reacted for 10 minutes. Then, 2M H₂SO₄ was added to the reaction solution in order to stop the reaction, and absorbance of the reaction solution was measured at a wavelength of 450 nm to measure the level of HRP activity.

As a result, it can be seen as the number of gold particles (hereinafter also referred to as the 'first solid support') that are linked with the labeled nucleic acid that is linked with the HRP labeled anti-DIG by bonding with DIG and the rat anti-PSA increases, HRP activity is linearly increased. Such results show that HRP activity is maintained in the manufacturing process using the gold particles.

### Example 3: Detection of target material using first solid support

In the present example, a prostate specific antigen (PSA) that constitutes a target material in a sample was detected using the first solid support produced according to Example 2.

First, a magnetic bead (DYNABEAD™ M-280 Tosylactivated, 2.8 µm) coated with a rat anti-PSA was prepared. The coating of the magnetic bead with the rat anti-PSA was performed using a method recommended by a manufacturer. Specifically, 1 ml of beads was subjected to a vortex motion for one minute and then collected by using a magnet for 2 minutes, and then mixed with 0.1 M borate buffer for 2 minutes. Then, the previous processes were performed once more, and then 0.1 M borate buffer was replaced with a rat anti-PSA dissolved in 0.1 M borate buffer and the reaction was performed at a temperature of 4°C for 20 hours.

100 µℓ of the rat anti-PSA coated magnetic bead (DYNABEAD™ M-280 Tosylactivated, 2.8µm) solution having a concentration of 2 x 10⁹ beads/ml, 20 µℓ of a sample including a varying concentration of PSA in a PBS buffer, and 100 µℓ of the first solid support solution having a concentration of 1x10⁹ particle/ml produced according to Example 2 were sufficiently reacted in a PBS buffer at 37 °C for 100 minutes, thereby forming a magnetic bead-PSA-first solid support composite. Then, the magnetic bead-PSA-first solid support composite was separated from the first solid support by magnetism. TMB that constitutes a substrate of HRP was added to the separated composite and then reacted for 10 minutes. 2M H₂SO₄ was added to the resultant reaction solution to stop the reaction and absorbance of the reaction solution was measured at a wavelength of 450 nm to measure the degree of HRP activity. Meanwhile, a control group was obtained by performing the same experiment as described above except that only the PBS buffer was used and PSA was not used.

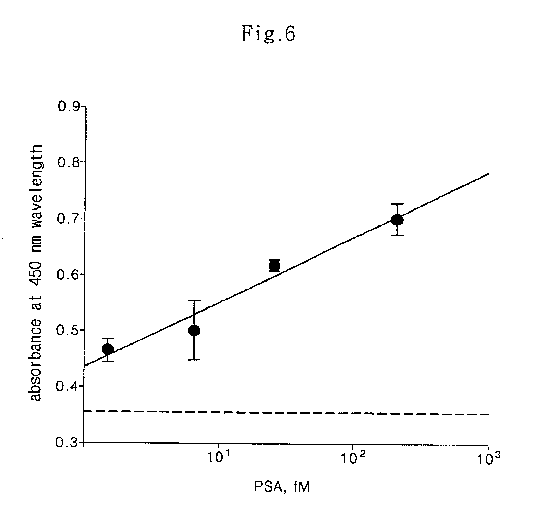

FIG. 6 is a diagram illustrating PSA assay results using a first solid support, with respect to the concentration of PSA in a sample. Referring to FIG. 6, a dotted line represents assay results of the control group, and a solid line represents assay results of the experimental group.

As illustrated in FIG. 6, PSA can be detected in as low a concentration as 1 fM or lower. Thus, it is confirmed that a target material can be detected with a high degree of sensitivity using a sold support as claimed.

As described above, in a solid support, a nucleic acid can be labeled with a high density of signal material.

A kit includes a fist solid support including a nucleic acid that is labeled with a high density of signal material. Accordingly, the kit can be efficiently used to assay a target material.

In a method of detecting a target material according to an, the target material can be assayed with a high degree of sensitivity.

## Claims

1. A solid support comprising a plurality of labeled nucleic acid molecules,
wherein the labeled nucleic acid molecules are immobilized on a surface of the solid support; and
wherein the labeled nucleic acid molecules comprise a plurality of nucleotide residues which are coupled to linking moieties,
wherein the solid support is further linked with a first target capture molecule that binds to a target material, and
wherein the length of the labeled nucleic acid molecules is in a range of 50 bp to 300 bp; wherein the labeled nucleic acid molecules comprise a region ("spacer region") ranging from the 5' end or 3' end thereof to the 30th position of their nucleotide sequence where no nucleotide residue is coupled to the linking moiety, and a region ("tail region") ranging from the 31 st position from the 5' end or 3' end to the other end of the molecule, where the nucleotide residues coupled to the linking moiety are present; and
wherein the labeled nucleic acid molecules are linked to the solid support through a free end of the spacer region.

2. The solid support of claim 1,
wherein the linking moiety is a material that emits a detectable signal.

3. The solid support of claim 2, wherein the material that emits a detectable signal is a fluorescent material or a radioactive material.

4. The solid support of claim 1,
wherein the linking moiety is linked to a linking partner molecule.

5. The solid support of claim 4,
wherein the linking partner molecule comprises a monoclonal antibody or fragments thereof that specifically binds the linking moiety, or
wherein the linking partner molecule is labeled with an enzyme.

6. The solid support of claim 4, wherein the linking moiety is selected from the group consisting of digoxygenin (DIG), dinitrophenol (DNP), and biotin, and the linking partner molecule is selected from the group consisting of anti-DIG antibody, anti-DNP antibody, and avidin or streptavidin, respectively.

7. The solid support of claim 5,
wherein the enzyme is selected from the group consisting of horseradish peroxidase (HRP), beta-galactosidase, glucuronidase and alkaline phosphatase (AP).

8. The solid support of claim 1,
wherein the nucleic acid molecule is selected from the group consisting of DNA, RNA, a chimera molecule of DNA and RNA, and a single-stranded nucleic acid.

9. The solid support of claim 1, wherein the solid support comprises a material selected from the group consisting of gold, polystyrene, polymethylmethacrylate (PMMA), nano particles thereof, and micro particles thereof.

10. The solid support of claim 1,
wherein the first target capture molecule comprises an antibody, a Fab fragment, or a nucleic acid that binds to the target material.

11. A method of detecting a target material in a sample, the method comprising:
bringing the sample into contact with a first solid support and a second solid support, wherein the first solid support is a solid support according to any one of claims 1 to 10, and the second solid support is linked with a second target capture molecule that binds to the target material;
isolating a composite of the target material, the first solid support, and the second solid support by centrifuging or using a magnet; and
detecting a signal generated by the isolated composite target material.

12. The method of claim 11,
wherein the linking moiety of the first solid support comprises a signal material that generates a detectable signal, and
wherein the detecting is performed by measuring the detectable signal generated by the signal material.

13. The method of claim 11,
wherein the linking moiety of the first solid support is linked to a linking partner molecule, and wherein the detecting may be performed by directly measuring a signal generated by the linking partner molecule, or by measuring a signal generated by an action of the linking partner molecule on its substrate, said substrate generates a detectable signal upon the action of the linking partner molecule.

14. The method of claim 11, wherein the first solid support is linked with a first target capture molecule, and the first target capture molecule and the second target capture molecule have different binding specificities.

## Patentansprüche

1. Feste Unterlage, umfassend eine Vielzahl markierter Nucleinsäuremoleküle, wobei die markierten Nucleinsäuremoleküle auf einer Oberfläche der festen Unterlage immobilisiert sind; und
wobei die markierten Nucleinsäuremoleküle eine Vielzahl Nucleotidreste umfassen, die an verknüpfende Einheiten gekoppelt sind,
wobei die feste Unterlage ferner mit einem ersten Ziel-Fängermolekül verknüpft ist, das an ein Zielmaterial bindet, und
wobei die Länge der markierten Nucleinsäuremoleküle in einem Bereich von 50 bp bis 300 bp liegt; wobei die markierten Nucleinsäuremoleküle eine Region ("Abstandsregion"), die von deren 5'-Ende oder 3'-Ende zu der 30. Position ihrer Nucleotidsequenz reicht, wo kein Nucleotidrest an die verknüpfende Einheit gekoppelt ist, und eine Region ("Schwanzregion"), die von der 31. Position vom 5'-Ende oder 3'-Ende zum anderen Ende des Moleküls reicht, wo die an die verknüpfende Einheit gekoppelten Nucleotidreste vorliegen, umfassen; und wobei die markierten Nucleinsäuremoleküle durch ein freies Ende der Abstandsregion mit der festen Unterlage verknüpft sind.

2. Feste Unterlage nach Anspruch 1,
wobei es sich bei der verknüpfenden Einheit um ein Material handelt, das ein nachweisbares Signal ausstrahlt.

3. Feste Unterlage nach Anspruch 2, wobei es sich bei dem Material, das ein nachweisbares Signal ausstrahlt, um ein fluoreszierendes Material oder ein radioaktives Material handelt.

4. Feste Unterlage nach Anspruch 1,
wobei die verknüpfende Einheit mit einem verknüpfenden Partnermolekül verknüpft ist.

5. Feste Unterlage nach Anspruch 4,
wobei das verknüpfende Partnermolekül einen monoklonalen Antikörper, der die verknüpfende Einheit spezifisch bindet, oder Fragmente davon umfasst oder wobei das verknüpfende Partnermolekül mit einem Enzym markiert ist.

6. Feste Unterlage nach Anspruch 4, wobei die verknüpfende Einheit aus der Gruppe ausgewählt ist, bestehend aus Digoxigenin (DIG), Dinitrophenol (DNP) und Biotin, und das verknüpfende Partnermolekül aus der Gruppe ausgewählt ist, bestehend aus anti-DIG-Antikörper, anti-DNP-Antikörper und Avidin bzw. Streptavidin.

7. Feste Unterlage nach Anspruch 5,
wobei das Enzym aus der Gruppe ausgewählt ist, bestehend aus Meerrettichperoxidase (HRP), beta-Galaktosidase, Glucuronidase und alkalischer Phosphatase (AP).

8. Feste Unterlage nach Anspruch 1,
wobei das Nucleinsäuremolekül aus der Gruppe ausgewählt ist, bestehend aus DNA, RNA, einem chimären Molekül aus DNA und RNA, und einer einzelsträngigen Nucleinsäure.

9. Feste Unterlage nach Anspruch 1, wobei die feste Unterlage ein Material umfasst, ausgewählt aus der Gruppe, bestehend aus Gold, Polystyrol, Polymethylmethacrylat (PMMA), Nanopartikeln davon und Mikropartikeln davon.

10. Feste Unterlage nach Anspruch 1,
wobei das erste Ziel-Fängermolekül einen Antikörper, ein Fab-Fragment oder eine Nucleinsäure umfasst, der/das/die an das Zielmaterial bindet.

11. Verfahren zum Nachweisen eines Zielmaterials in einer Probe, wobei das Verfahren umfasst:
In-Kontakt-Bringen der Probe mit einer ersten festen Unterlage und einer zweiten festen Unterlage, wobei es sich bei der ersten festen Unterlage um eine feste Unterlage gemäß einem der Ansprüche 1 bis 10 handelt, und die zweite feste Unterlage mit einem zweiten Ziel-Fängermolekül verknüpft ist, das an das Zielmaterial bindet;
Isolieren eines Verbunds des Zielmaterials, der ersten festen Unterlage und der zweiten festen Unterlage durch Zentrifugieren oder Verwenden eines Magneten; und
Nachweisen eines durch das isolierte Verbund-Zielmaterial erzeugten Signals.

12. Verfahren nach Anspruch 11,
wobei die verknüpfende Einheit der ersten festen Unterlage ein Signalmaterial umfasst, das ein nachweisbares Signal erzeugt, und
wobei das Nachweisen durch Messen des durch das Signalmaterial erzeugten nachweisbaren Signals durchgeführt wird.

13. Verfahren nach Anspruch 11,
wobei die verknüpfende Einheit der ersten festen Unterlage mit einem verknüpfenden Partnermolekül verknüpft ist und wobei das Nachweisen durch direktes Messen eines durch das verknüpfende Partnermolekül erzeugten Signals oder durch Messen eines durch eine Aktivität des verknüpfenden Partnermoleküls an seinem Substrat erzeugten Signals durchgeführt wird, wobei das Substrat auf die Aktivität des verknüpfenden Partnermoleküls hin ein nachweisbares Signal erzeugt.

14. Verfahren nach Anspruch 11, wobei die erste feste Unterlage mit einem ersten Ziel-Fängermolekül verknüpft ist und das erste Ziel-Fängermolekül und das zweite Ziel-Fängermolekül unterschiedliche Bindungsspezifitäten aufweisen.

## Revendications

1. Support solide comprenant une pluralité de molécules d'acide nucléique marquées,
où les molécules d'acide nucléique marquées sont immobilisées sur une surface du support solide ; et
où les molécules d'acide nucléique marquées comprennent une pluralité de résidus nucléotidiques qui sont couplés à des fragments de liaison,
où le support solide est en outre lié à une première molécule de capture de cible qui se lie à un matériau cible, et
où la longueur des molécules d'acide nucléique marquées se situe dans une plage de 50 bp à 300 bp ;
où les molécules d'acide nucléique marquées comprennent une région ("région intercalaire") allant de l'extrémité 5' ou de l'extrémité 3' de celles-ci jusqu'à la 30^{ème} position de leur séquence nucléotidique où aucun résidu nucléotidique n'est couplé au fragment de liaison, et une région ("région de queue") allant de la 31^{ème} position à partir de l'extrémité 5' ou de l'extrémité 3' jusqu'à l'autre extrémité de la molécule où sont présents les résidus nucléotidiques couplés au fragment de liaison ; et
où les molécules d'acide nucléique marquées sont liées au support solide par l'intermédiaire d'une extrémité libre de la région intercalaire.

2. Support solide selon la revendication 1,
où le fragment de liaison est un matériau qui émet un signal détectable.

3. Support solide selon la revendication 2, où le matériau qui émet un signal détectable est un matériau fluorescent ou un matériau radioactif.

4. Support solide selon la revendication 1,
où le fragment de liaison est lié à une molécule partenaire de liaison.

5. Support solide selon la revendication 4,
où la molécule partenaire de liaison comprend un anticorps monoclonal ou des fragments de celui-ci qui se lie de manière spécifique au fragment de liaison, ou
où la molécule partenaire de liaison est marquée avec un enzyme.

6. Support solide selon la revendication 4,
où le groupement de liaison est sélectionné parmi le groupe constitué de la digoxygénine (DIG), du dinitrophénol (DNP) et de la biotine, et la molécule partenaire de liaison est sélectionnée parmi le groupe constitué de l'anticorps anti-DIG, de l'anticorps anti-DNP, et de l'avidine ou de la streptavidine, respectivement.

7. Support solide selon la revendication 5,
où l'enzyme est sélectionné parmi le groupe constitué de la peroxydase de raifort (HRP), de la bêta-galactosidase, de la glucuronidase et de la phosphatase alcaline (PA).

8. Support solide selon la revendication 1,
où la molécule d'acide nucléique est sélectionnée parmi le groupe constitué de l'ADN, de l'ARN, d'une molécule chimérique d'ADN et d'ARN, et d'un acide nucléique monocaténaire.

9. Support solide selon la revendication 1, où le support solide comprend un matériau sélectionné parmi le groupe constitué de l'or, du polystyrène, du polyméthylméthacrylate (PMMA), des nanoparticules de ceux-ci et des microparticules de ceux-ci.

10. Support solide selon la revendication 1,
où la première molécule de capture de cible comprend un anticorps, un fragment Fab, ou un acide nucléique qui se lie au matériau cible.

11. Procédé de détection d'un matériau cible dans un échantillon, le procédé comprenant :
une mise en contact de l'échantillon avec un premier support solide et un second support solide,
où le premier support solide est un support solide selon l'une quelconque des revendications 1 à 10, et le second support solide est lié à une seconde molécule de capture de cible qui se lie au matériau cible ;
un isolement d'un composite du matériau cible, du premier support solide et du second support solide par centrifugation ou utilisation d'un aimant ; et
une détection d'un signal généré par le matériau cible composite isolé.

12. Procédé selon la revendication 11,
où le fragment de liaison du premier support solide comprend un matériau de signal qui génère un signal détectable, et
où la détection est réalisée en mesurant le signal détectable généré par le matériau de signal.

13. Procédé selon la revendication 11,
où le fragment de liaison du premier support solide est lié à une molécule partenaire de liaison, et où la détection peut être réalisée en mesurant directement un signal généré par la molécule partenaire de liaison, ou en mesurant un signal généré par une action de la molécule partenaire de liaison sur son substrat, ledit substrat générant un signal détectable lors de l'action de la molécule partenaire de liaison.

14. Procédé selon la revendication 11, où le premier support solide est lié à une première molécule de capture de cible, et la première molécule de capture de cible et la seconde molécule de capture de cible présentent des spécificités de liaison différentes.
